Europäisches Patentamt

European Patent Office

Office européen des brevets

(11) Numéro de publication: **0 429 316 A1**

# DEMANDE DE BREVET EUROPEEN

(21) Numéro de dépôt: 90402869.3

(22) Date de dépôt: **15.10.90**

(51) Int. Cl.⁵: **C07D 317/54**

(30) Priorité: 02.11.89 FR 8914358

(43) Date de publication de la demande:
**29.05.91 Bulletin 91/22**

(84) Etats contractants désignés:
**AT BE CH DE DK ES FR GB GR IT LI LU NL SE**

(71) Demandeur: **SOCIETE FRANCAISE HOECHST TOUR ROUSSEL HOECHST 1 Terrasse Bellini F-92800 Puteaux(FR)**

(72) Inventeur: **Sidot, Christian**
**Résidence des Fleurs, Avenue Foch**
**F-95460 Ezanville(FR)**
Inventeur: **Christidis, Yani**
**53, Boulevard de la Villette**
**F-75019 Paris(FR)**

(74) Mandataire: **Rinuy, Santarelli**
**14, avenue de la Grande Armée**
**F-75017 Paris(FR)**

(54) **Procédé de préparation du pipéronal.**

(57) Procédé de préparation du pipéronal par oxydation décarboxylante de l'acide méthylènedioxy-3,4 mandélique par de l'acide nitrique en milieu aqueux.

EP 0 429 316 A1

# PROCÉDÉ DE PRÉPARATION DU PIPÉRONAL.

La présente invention concerne un procédé de préparation de pipéronal.

Le pipéronal, connu en parfumerie sous le nom d'héliotropine, peut être obtenu soit par chauffage de l'aldéhyde protocatéchique avec de la potasse et de l'iodure de méthylène, soit par application de la réaction de Sommelet au chlorure de pipéronyle, soit par oxydation de l'acide pipérique, du safrole ou mieux de l'isosafrole par le permanganate de potassium ou l'ozone, soit enfin par oxydation décarboxylante de l'acide méthylènedioxy-3,4 mandélique par le complexe benzoate de l'argent-iode au reflux du benzène (Beilstein, 19, 115,119, I, 660-662, II, 141-143, et P.S. Raman Current Science, 1958, 27, 22) ou à 100° C par une quantité équivalente d'acide nitrique dilué dans de l'eau.

On sait que les acides carboxyliques alphahydroxylés peuvent être dégradés en leur dérivé carbonylé correspondant soit par simple chauffage, soit par action de l'acide sulfurique, soit par oxydation. La thermolyse est surtout applicable aux acides gras avec formation intermédiaire d'un lactide. Le clivage sulfurique semble limité aux acides citriques et mélique. Quant aux scissions oxydantes, elles font généralement appel à l'iode ou à ses complexes comme dans le cas de la dégradation de l'acide mandélique en benzaldéhyde (Annales, 1926, 446, 71) ou de l'acide méthylènedioxy-3,4 mandélique en pipéronal (P.S. Raman, loc. cit. ) ou à des agents oxydants adaptés aux clivages de groupements fonctionnels vicinaux tels que le tétraacétate de plomb.

On constate donc que les procédés connus d'obtention de pipéronal ne sont pas économiquement satisfaisants en raison de l'emploi de réactifs ou de matières premières coûteux ou de la mise en oeuvre de réactions difficilement extrapolables à l'échelle in dustrielle. Devant la demande de plus en plus importante d'héliotropine pure, il était donc nécessaire de pouvoir disposer d'un procédé industriel économique pour l'obtenir.

Or la demanderesse vient de découvrir un nouveau procédé pour accéder facilement et avec de bons rendements au pipéronal à partir de l'acide méthylènedioxy-3,4 mandélique. Le procédé de la présente invention consiste à dégrader l'acide méthylènedioxy-3,4 mandélique en pipéronal par oxydation décarboxylante et il est caractérisé en ce que cette oxydation est réalisée en milieu aqueux par de l'acide nitrique en présence d'acide chlorhydrique.

L'acide chlorhydrique accélère et surtout permet de régler très aisément la vitesse d'oxydation décarboxylante de l'acide méthylènedioxy-3,4 mandélique par l'acide nitrique. Avantageusement, on utilise entre 1 et 2 moles d'acide chlorhydrique par mole d'acide méthylènedioxy-3,4 mandélique mis en oeuvre.

La quantité d'acide nitrique mis en oeuvre est de préférence voisine de la quantité stoechiométrique, c'est-à-dire voisine de 0,66 mole d'acide nitrique par mole d'acide méthylènedioxy-3,4 mandélique mis en oeuvre. On opère notamment en léger excès d'acide nitrique, à environ 0,8 mole d'acide nitrique par mole d'acide méthylènedioxy-3,4 mandélique. L'acide nitrique utilisé est un acide nitrique commercial de concentration comprise entre 65 et 69 % et il est introduit lentement au fur et à mesure de sa consommation dans une suspension aqueuse d'acide méthylènedioxy-3,4 mandélique. La réaction d'oxydation décarboxylante de l'acide méthylènedioxy-3,4 mandélique est exothermique, si bien que l'on règle la vitesse d'introduction de l'acide nitrique dans la suspension aqueuse d'acide méthylènedioxy-3,4 mandélique de manière à maintenir la température désirée sans l'aide d'un chauffage ou d'un refroidissement externes. Afin de faciliter le démarrage de la réaction d'oxydation, il est parfois avantageux d'introduire dans le milieu réactionnel une très faible quantité de nitrite de sodium. Dans ce cas, on peut utiliser 1 mmole de nitrite de sodium par mole d'acide méthylènedioxy-3,4 mandélique mis en oeuvre.

Le procédé selon la présente invention est réalisé avantageusement à une température comprise entre la température ambiante et 50° c, préférentiellement entre 40° et 50° C, et il est conduit en milieu aqueux. Etant donné que l'acide méthylènedioxy-3,4 mandélique ainsi que le pipéronal sont des produits très peu solubles dans l'eau, le procédé selon l'invention s'effectue généralement en phase hétérogène. En fin de réaction, le pipéronal formé est isolé du milieu réactionnel par des moyens connus en soi. Avantageusement, il est extrait à l'aide d'un solvant compatible non miscible à l'eau, puis, si nécessaire, il est purifié soit par distillation sous pression réduite, soit par recristallisation dans l'éthanol à 70 % dans l'eau.

L'exemple suivant illustre la présente invention sans toutefois la limiter.

## Exemple

On mélange sous agitation à la température ambiante :
- 294,23 g (1,5 mole) d'acide méthylènedioxy-3,4 mandélique ;
- 562 g d'eau ;
- 258,75 g d'acide chlorhydrique à 37 %, soit 2,625 moles ;

- 21 g d'acide nitrique à 69 %, soit 23 mmoles.

la suspension obtenue est chauffée sous agitation à 43 + 2° C, puis à cette température, on introduit rapidement 103,5 mg (1,5 mole) de nitrite de sodium dissous dans 4 g d'eau, puis lentement, en 3 heures environ, de manière à maintenir la température du milieu réactionnel entre 40 et 50° C sans l'aide d'un chauffage ou d'un refroidissement externes, 107,5 g d'acide nitrique à 69 %, soit 1,177 mole. En fin d'introduction, on abandonne le milieu réactionnel une heure sous agitation à 43 + 2° C puison le refroidit à la température ambiante et enfin on l'extrait trois fois avec 600 g de trichloro-1,1,1-éthane. Les phases organiques réunies sont ensuite lavées successivement une fois à l'eau, trois fois avec une solution aqueuse saturée d'hydrogénocarbonate de sodium et enfin une fois à l'eau avant d'être concentrées sous pression réduite.

On isole ainsi 220 g (1,46 mole) de pipéronal brut que l'on purifie par distillation sous pression réduite.

On isole ainsi 178 g (1,186 mole) de pipéronal pur distillant à 106° C sous un vide de 2,4 mbars et présentant un point de fusion de 37 + 1° C. Le rendement s'établit à 79 % de la théorie calculée par rapport à l'acide méthylènedioxy-3,4 mandélique mis en oeuvre.

**Revendications**

1. Procédé de préparation du pipéronal par oxydation nitrique de l'acide méthylènedioxy-3,4 mandélique, caractérisé en ce que l'oxydation est effectuée en présence d'acide chlorydrique.

2. Procédé selon la revendication 1, caractérisé en ce que l'on utilise environ une quantité stoechiométrique d'acide nitrique.

3. Procédé selon l'une quelconque des revendications 1 et 2, caractérisé en ce qu'il est réalisé à une température comprise entre la température ambiante et 50° C.

4. Procédé selon l'une quelconque des revendications 1 à 3, caractérisé en ce qu'il est réalisé en phase hétérogène.

# RAPPORT DE RECHERCHE EUROPEENNE

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | CHEMICAL ABSTRACTS, vol. 112, 1990, page 700, résumé no. 55145n, Columbus, Ohio, US; L. CERVENY et al.: "Synthesis of heliotropin", & PERFUM. FLAVOR. 1989, 14(2), 13-14,16-18 * Résumé * | 1-3 | C 07 D 317/54 |

- - - - -

|  |  |  | DOMAINES TECHNIQUES RECHERCHES (Int. Cl.5) |
|---|---|---|---|
|  |  |  | C 07 D 317/00 |

Le présent rapport de recherche a été établi pour toutes les revendications

| Lieu de la recherche | Date d'achèvement de la recherche | Examinateur |
|---|---|---|
| La Haye | 25 février 91 | FRANCOIS J.C.L. |